# EUROPEAN PATENT APPLICATION

(11) **EP 4 656 695 A1**
(43) Date of publication of application: **03.12.2025**
(21) Application number: 25178106.8
(22) Date of filing: 22.05.2025
(51) Int. Cl.: C09J 5/06, A61M 5/34

(54) **METHOD OF CURING ADHESIVE WITH HOT AIR TREATMENT**

(30) Priority: 29.05.2024 US 202463652930 P
(71) Applicant: ATS Corporation, Cambridge, Ontario N3H 4R7 (CA)
(72) Inventor: LINDNER, Roland, 85551 Heimstetten (DE); KOHLMEIER, Christoph, 85551 Heimstetten (DE); DEKHIL, Hamdi, 85551 Heimstetten (DE)
(74) Representative: Bardehle Pagenberg Partnerschaft mbB Patentanwälte Rechtsanwälte

(57) **Abstract**

A method for curing adhesive during assembly of a glass syringe comprises the following steps a.) to d.): a.) providing an adhesive, a glass barrel and a needle; b.) dispensing the adhesive onto the glass barrel and/or needle; c.) arranging the needle onto the barrel; d.) curing the adhesive by means of radiation; and the method further comprises: heating at least one of the glass barrel, needle and adhesive by means of hot air.

## Description

### 1. Field of the invention

The present disclosure relates to a system and method for curing adhesives. More particularly, the present disclosure relates to a system and method for curing adhesive when assembling a glass syringe, in particular for pharmaceutical applications.

### 2. Background

Modern manufacturing and automation systems and processes are growing increasingly complex, driven by the need for speed, precision, and consistency to ensure high-quality products within shorter timeframes. These systems strive to achieve maximum efficiency and minimal downtime for maintenance and troubleshooting, while also maintaining low production costs per part. Additionally, there is an ongoing trend towards continuous improvement in these areas to adapt to the evolving manufacturing landscape.

A particular type of automation involves the assembly of glass syringes, where a cannula is attached to a glass barrel or hub using adhesive. In pharmaceutical applications, it is crucial to prevent contamination of the syringe surfaces, as this could threaten the sterility of any drugs stored within. Regulatory and standards organizations therefore dictate the specific adhesives that can be used, such as Loctite^{™} 3345, which has gained market approval for its safety in drug packaging. This adhesive not only proves harmless to patients but also remains compatible with other packaging materials throughout the product's shelf life. It is therefore commonly employed in assembling glass syringes. The adhesive is typically cured using a mercury lamp, although this method is both inefficient and costly. Additionally, due to environmental concerns, the use of mercury lamps is gradually being discontinued.

WO 2020/118456 A1 discloses a method for curing adhesive during assembly of glass syringes, including: dispensing adhesive onto a glass syringe; and cur-ing the adhesive using a UV LED line array. The method may include one or both of pre-curing the adhesive using a UV LED spot and heating at least one of the glass barrel, needle and adhesive at one or more points during the method. With this method, the curing of the adhesive is not optimal due to oxygen inhibition. Infrared radiation will heat the adhesive from the inside, while hot air treatment will heat the adhesive from the outside.

It is disadvantageously here, that some of the adhesive partially remains not crosslinked on the outer surface of the adhesive, as oxygen can attach to the polymers and inhibit curing. Thus, there is a demand for an improved adhesive treatment that addresses the effect of oxygen inhibition and supplies an optimized relation of varying hot air and radiation treatment.

In view of the foregoing, there is a need for improved systems and methods for curing adhesive, particularly in operations such as assembly of (pharmaceutical) glass syringes. It is thus an object of the present invention to overcome some or all the deficiencies of the prior art and it is a particular object of the present invention to provide a method that allows a combination of LED light curing and hot air treatment for an improved adhesive treatment.

### 3. Summary

The above objects are at least partially achieved by the subject matter of independent claim 1. Preferred embodiments are the subject of the dependent claims, and the skilled person will find clues to other suitable aspects of the present invention in the overall disclosure of the present application.

An aspect of the invention relates to a method for curing adhesive during assembly of a (preferably pharmaceutical) glass syringe, the method comprising the following steps a.) to d.) (preferably in the given order): a.) providing an adhesive, a glass barrel and a needle; b.) dispensing the adhesive onto the glass barrel and/or needle; c.) arranging the needle onto the barrel; d.) curing the adhesive by means of radiation; and wherein the method further comprises: heating at least one of the glass barrel, needle and adhesive by means of hot air.

This process optimizes the assembly of glass syringes by enhancing the bonding strength and durability of the adhesive connection between the needle and the barrel. By heating with hot air, the curing process can be accelerated and made more efficient, ensuring that the adhesive fully sets without compromising the integrity of the glass syringe. Hot air treatment provides stimulation, especially on the surface of the adhesive. The adhesive is heated from the outside to the inside. This method is particularly beneficial in pharmaceutical applications where precise and reliable assembly of glass syringes is critical. This technique offers an advantage in improving the overall quality and safety of the syringes, potentially reducing defects and failures in medical applications.

The invention can be furtherly embodied, when the heating is carried out during and/or after step d.).

In one preferred embodiment, this refined approach allows for greater control over the adhesive's curing process. By applying heat during or after the radiation-induced curing, it helps to further ensure that the adhesive sets completely and uniformly. This method can be crucial in scenarios where the adhesive needs more energy than the radiation alone can provide, or when a post-curing temperature treatment is necessary to achieve optimal bonding properties.

The invention can be improved, when the heating is carried out with hot air and UV radiation, preferably provided by a LED.

The process is suitable for all UV-curable adhesives with acrylate chemistry. In particular, for adhesives with an adhesive receptive to oxygen inhibition. In preferable or alternative embodiments, the adhesive can be selected from but not limited to: Henkel Loctite AA 3345^{®}, Loctite AA 3922^{®}, Loctite AA 3943^{®}, Loctite AA 3321^{®}, Loctite AA 3311^{®}, Loctite AA 3301^{®}. The inclusion of UV radiation, especially from LEDs, enhances the curing process by providing a specific wavelength of light that can be more effective in activating certain adhesives. LEDs offer a consistent and controllable source of UV radiation, which can be adjusted to match the optimal curing characteristics for different types of adhesives used in syringe assembly. This may be followed by again providing hot air and after that UV radiation, which may be repeated several times.

In another embodiment of the invention, the hot air and the UV radiation are used alternately.

The alternating use of hot air and UV radiation optimizes the curing process by leveraging the distinct benefits of each heating method. Initially using hot air can rapidly elevate the temperature of the adhesive, glass barrel, and needle to near-optimal curing conditions. Following this with UV radiation, especially from LEDs, can then provide a more targeted energy input, precisely tuned to the adhesive's requirements for a complete cure.

In a preferred embodiment, the hot air is provided in an angle of incidence in the range 0° to 60°, preferably in the range 15° to 45°, most preferred about 30°.

The directed application of hot air at these specific angles ensures that the heat is distributed more evenly and effectively around the adhesive and the components being bonded. The choice of these parameters is particularly effective for achieving a focused application of heat that penetrates the adhesive without causing overheating or damage to the delicate glass structure of the syringe.

In a preferred embodiment, the radiation is provided in an angle of incidence in the range 0° to 60°, preferably in the range 15° to 45°, most preferred around 30°.

Applying radiation at these specified angles ensures that the energy is optimally distributed across the adhesive area. The 30° angle, in particular, is especially advantageous for focusing the radiation to penetrate the adhesive effectively while avoiding direct exposure to the sensitive areas of the glass barrel and needle that could potentially cause damage or degradation.

The method is improved, when the radiation is provided by a radiation source, and the shortest distance between the radiation source and the adhesive is in the range between 10 mm and 40 mm, preferably in the range 20 mm to 30 mm, most preferably around 26 mm.

Setting the radiation source at these specific distances ensures that the radiation energy is optimally focused on the adhesive. At these parameters the radiation can effectively penetrate and cure the adhesive without overexposure, which could potentially degrade the materials or cause uneven curing. This distance is particularly effective in optimizing the intensity and coverage of the radiation, ensuring that it reaches the adhesive adequately for a strong and uniform bond.

The method can be improved, when the hot air is provided by an air-nozzle, and the shortest distance between the air-nozzle and the adhesive is in the range between 5 mm and 20 mm, preferably in the range 7 mm to 15 mm, most preferably around 10 mm.

Positioning the air-nozzle at these distances ensures optimal thermal management during the curing process. At these parameters the hot air can effectively reach the adhesive to accelerate the curing process while avoiding overheating or damaging the glass barrel or the needle. This precise distance allows for efficient heat transfer to the adhesive, ensuring that it is evenly heated and cured without creating thermal gradients that could lead to inconsistencies in the bond strength.

To improve the method further, the hot air is provided in the vicinity of the glass barrel, needle and/or adhesive with a temperature in the range of between 150 °C to 450°C, preferably between 200 °C and 400°C, more preferably between 220 °C and 300 °C, most preferably around 250 °C.

Using hot air at these parameters ensures that the adhesive is rapidly and evenly cured. This temperature is high enough to effectively accelerate the curing process but not so high as to risk damaging the sensitive materials of the glass barrel or the needle. The control of temperature within this specific range helps in achieving a strong and durable bond while maintaining the integrity and functionality of the syringe components.

Further improvement of the invention is achieved, when the wavelength of the radiation is in the range 320 nm to 410 nm, preferably in the range 340 nm to 390 nm, most preferably around 365 nm.

Employing radiation at these parameters, which typically falls within the UVA spectrum, is particularly effective for curing adhesives commonly used in medical applications. This wavelength is well-suited to initiate the polymerization processes in many photo-reactive adhesives, leading to a strong and durable bond between the needle and the glass barrel.

To improve the method further, the intensity of the radiation is in the range 1500 mW/cm² to 7000 mW/cm², preferably in the range 2500 mW/cm² to 6000 mW/cm², most preferably around 3500 mW/cm².

Utilizing radiation at an intensity of these parameters ensures that the adhesive receives sufficient energy to trigger and complete the curing process effectively and efficiently. This intensity is carefully calibrated to ensure that it is strong enough to thoroughly cure the adhesive, yet not so intense as to risk damaging the glass syringe components or overheating the adhesive, which could result in degradation or inadequate bonding.

In another embodiment of the invention, the hot air is provided by an air-nozzle with a flow rate per cm² of the nozzle exit in the range of 60 l/min to 250 l/min, preferably 80 l/min to 200 l/min, more preferably 100 l/min to 150 l/min, most preferably around 125 l/min. Thus, if the nozzle exit has e.g., a cross section of 10 cm², a flow rate of e.g., 60 l/min would correspond to a total flow rate of 600 l/min flowing from the nozzle exit.

Delivering hot air at a flow rate of these parameters ensures that the heat is evenly distributed across the adhesive and the components being bonded, without causing displacement of the adhesive. This rate is optimal for providing sufficient heat to aid the curing process effectively while maintaining the structural integrity of both the adhesive and the syringe components.

This embodiment can be further improved, when the flow rate during the heating process is varied over time.

The initial phase might use a higher flow rate to quickly elevate the temperature of the adhesive and surrounding materials to a suitable level for curing. As the curing process progresses, the flow rate could be reduced to maintain the required temperature or to avoid overheating, which could degrade the adhesive or damage the glass syringe components. This dynamic adjustment of the flow rate helps in managing the thermal profile during the curing process more effectively.

The invention can be further improved, when the heating is carried out for a duration between 4 and 15 seconds, preferably between 6 and 12 seconds, more preferably between 7 and 10 seconds, most preferably around 8 seconds.

Applying the heating with these parameters provides a balanced approach that is long enough to effectively activate and set the adhesive, yet short enough to prevent any thermal damage to the glass syringe components or degradation of the adhesive quality. This precise timing helps to ensure that the adhesive cures uniformly, forming a strong and durable bond between the needle and the glass barrel.

The method according to the invention is improved, when the radiation is provided for a duration between 4 and 15 seconds, preferably between 6 and 12 seconds, more preferably between 7 and 10 seconds, most pref-erably around 8 seconds.

Using radiation with these parameters optimizes the curing process. This duration is carefully calibrated to provide enough energy to properly activate and set the adhesive, while preventing any potential damage to the sensitive materials of the glass barrel or needle from prolonged exposure. This controlled exposure ensures that the adhesive achieves a strong, durable bond essential for the structural integrity of the glass syringe.

Further improvement of the method is achieved when the surface of the adhesive is dry after the heating.

Achieving a dry surface on the adhesive signifies that the adhesive has undergone full polymerization, forming a strong, solid bond between the glass barrel and the needle.

The invention can further be improved when the temperature during the heating is varied over time.

The initial phase of the heating might involve a higher temperature to rapidly bring the adhesive up to a suitable reaction temperature, followed by a gradual decrease to prevent overheating as the adhesive begins to set. Alternatively, the temperature might be gradually increased to a peak before being reduced, ensuring that the adhesive reaches and maintains the optimal temperature long enough to achieve complete curing without degradation.

Further improvement to this embodiment is obtained when the temperature is steadily decreased or increased over time.

The advantage of this method, whether steadily increasing or steadily decreasing temperature, lies in its ability to provide a more controlled approach to heating, optimizing the adhesive's curing conditions throughout the process.

This embodiment can be improved when the temperature variation is done with a gradient ΔT/Δt in the range of from 5 K/min to 50 K/min, preferably from 10 K/min to 40 K/min, more preferably from 20 K/min to 30 K/min.

A gradient with these parameters allows for a controlled yet efficient rate of temperature change, providing a balanced approach that can quickly bring the adhesive up to the necessary curing temperature without causing thermal degradation.

Another embodiment of the invention is obtained, when the heating is carried out such that the adhesive during the heating has a surface temperature in the range of between 100 °C to 350°C, preferably between 150 °C and 300°C, more preferably between 200 °C and 250 °C.

Maintaining the adhesive's surface temperature within the range be-tween with these parameters ensures that the adhesive cures effectively without reaching temperatures that could degrade its chemical structure or compromise its bonding strength. This temperature range is carefully chosen to activate and sustain the curing reactions necessary for the adhesive to achieve a strong and durable bond between the glass barrel and the needle.

A further embodiment of the invention is achieved, when before step b.) and/or during step c.) at least one of the glass barrel, needle and adhesive is provided with heat.

Heating the glass barrel or the needle before applying the adhesive ensures that the surface temperature of these components is optimal for adhesive application, enhancing the adhesive's bonding properties. Similarly, heating during the assembly process, specifically when arranging the needle onto the barrel, can facilitate a faster and more uniform cur-ing of the adhesive by maintaining the assembly at a consistent temperature conducive to curing.

Another embodiment of the invention is achieved, when after step b.) and before step d.) the adhesive is pre-cured.

The pre-curing process might involve exposing the adhesive to a lower intensity or shorter duration of radiation or heat compared to the main curing process. This preliminary treatment helps to begin the chemical reactions needed for curing without completing them, enabling the adhesive to reach a state where it holds the components e.g., glass barrel and needle in place but remains sufficiently malleable for final positioning adjustments if necessary.

A further embodiment of the invention is achieved, when the curing comprises irradiating the adhesive with an LED line array.

The LED line array emits light at wavelengths specifically suited to activate the curing process of the particular adhesive used. These wavelengths are generally in the ultraviolet (UV) range, which is effective for initiating photochemical reactions in UV-curable adhesives. The line array allows for even distribution of light, ensuring that all parts of the adhesive receive uniform exposure, which is critical for achieving consistent curing throughout the joint area between the glass barrel and the needle.

### 4. Brief description of the figures

In the following, preferred embodiments of the disclosure are disclosed by reference to the accompanying figures.
- Fig. 1:: illustrates the treatment of syringes in an alternating arrangement of LED curing and hot air treatment in a perspective view.
- Fig. 2:: shows the hot air treatment of a syringe in a side view.
- Fig. 3:: shows the LED curing of the adhesive on a syringe in a side view.
- Fig. 4:: illustrates the syringe comprising needle, glass barrel and adhesive in detail.

### 5. Detailed description of the figures

The subsequent sections provide a detailed description of the invention, referencing the accompanying illustrations for clarity. The descriptions represent examples only and are not intended to limit the invention's scope. Identical reference numerals across the figures and text denote the same components. The illustrations may not reflect actual size or scale; their dimensions, proportions, and depictions of elements might be enhanced for better understanding and visual convenience.

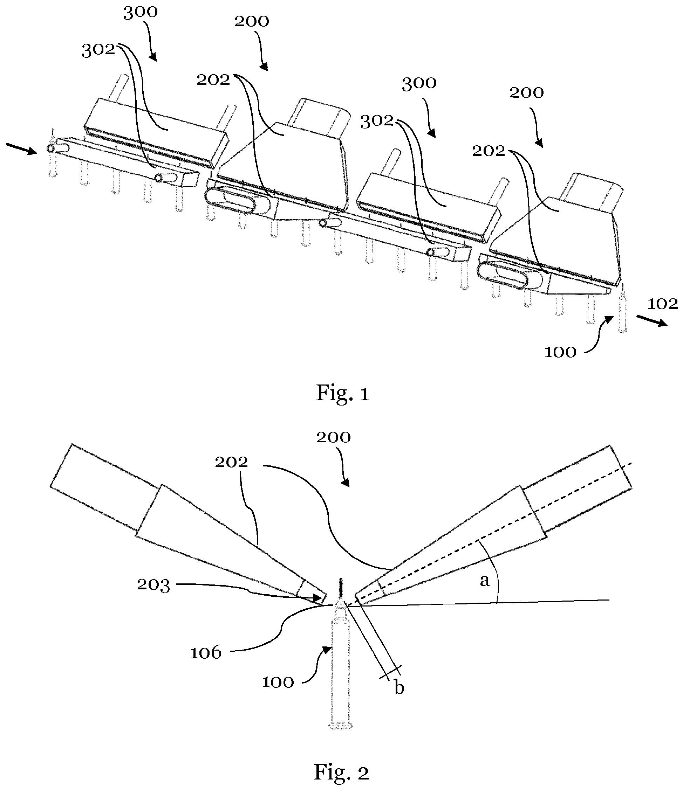

Figure 1 illustrates an embodiment of the invention, where syringes 100 are moved in a direction 102 on a production line. Upon moving in the direction 102, the syringes 100 pass hot air treatment stations 200 and UV radiation treatment stations 300. In the shown embodiment, a UV radiation treatment station 300 comprises two LED floodlights 302 and a hot air treatment station 200 comprises two air nozzles 202. The shown production line comprises two UV radiation treatment stations 300 and two hot air treatment stations 200. The UV radiation treatment stations 300 and the hot air treatment stations 200 are arranged alternating, so that a syringe 100 moving through the production line in the direction 102 is alternately exposed to heat and radiation.

The air nozzles 202 direct hot air towards the syringes 100 to either preheat them before application of the adhesive 106, facilitate drying of the adhesive 106, or enhance the curing process by maintaining optimal temperatures. The UV floodlights 302 emit UV radiation that is necessary for curing the adhesive 106 applied between the glass barrel 104 and the needle 108 of the syringe. The UV radiation promotes rapid and effective polymerization of the adhesive. This arrangement ensures that as syringes 100 move along the line, they are sequentially exposed to alternating treatments of heat and UV radiation. This alternating exposure pattern is designed to optimize the curing process. The hot air potentially pre-cures or maintains the temperature of the adhesive, making it more receptive to the subsequent UV curing step. The UV radiation then provides a strong, focused energy source to complete the cur-ing process, ensuring a durable bond.

Figure 2 depicts a side view of a hot air treatment station 200, comprising two air nozzles 202 positioned at the left and right of a syringe 100. The air nozzles 202 are arranged at an angle a to the syringe 100 and with a distance b to the surface of the adhesive 106. The air nozzles 202 have a nozzle exit 203 with an exit area of e.g., 5 cm². A suitable air flow rate is e.g., 100 l/min per cm² of the nozzle exit, corresponding to a total flow rate of 500 l/min in this example.

The angle a is important as it determines the direction and focus of the hot air stream from the air nozzle 202 towards the adhesive 106. Setting the air nozzles 202 at an optimal angle a ensures that the hot air effectively reaches and uniformly heats the adhesive without causing damage to other parts of the syringe. The distance b is the distance from the air nozzle 202 to the surface of the adhesive 106 on the syringe 100. This distance is essential to ensure that the hot air affects the adhesive 106 effectively, promoting optimal curing conditions without overheating. The distance b needs to be close enough to positively impact the adhesive but far enough to prevent any thermal damage for example to the glass barrel 104, the needle 108 or the adhesive 106.

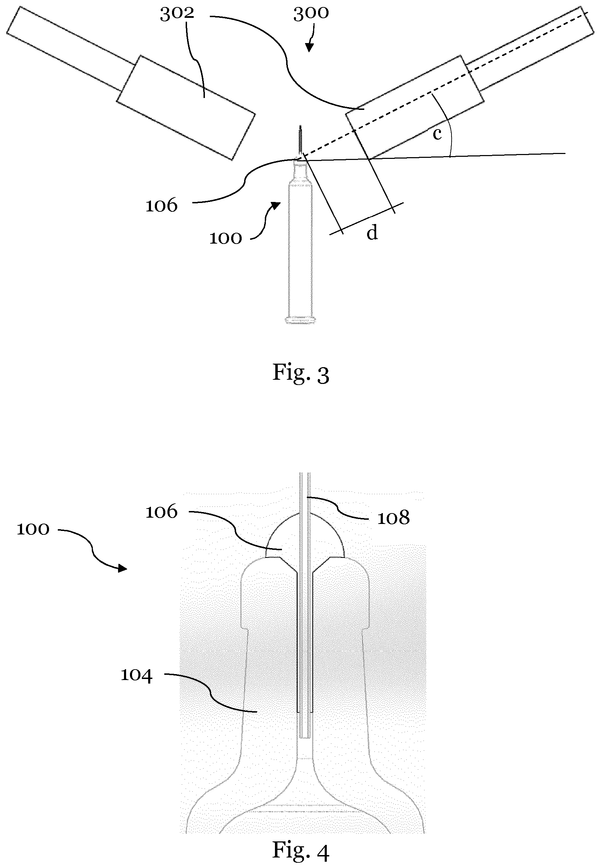

Figure 3 illustrates a side view of a UV radiation treatment station 300, comprising two LED floodlights 302 positioned at the left and right of a syringe 100. The LED floodlights 302 are arranged at an angle c to the syringe 100 and with a distance d to the surface of the adhesive 106.

The angle c is important for ensuring that the UV light effectively targets the adhesive area 106 and ensures that the UV radiation is focused and uniform, maximizing the curing effect while minimizing exposure to non-targeted areas. The distance d is the distance from each LED floodlight 302 to the surface of the adhesive 106 on the syringe 100. The correct distance is vital for ensuring optimal radiation levels reach the adhesive 106. If the LED floodlights 302 are too close, they might cause overheating or uneven curing. If too far, the intensity might not be sufficient to cure the adhesive 106 effectively.

Figure 4 depicts a syringe 100 in a detailed close-up view. The syringe comprises a glass barrel 104, an adhesive 106 and a needle 108. The adhesive 106 holds the needle 108 at a defined position in the glass barrel 104. The needle 108 is aligned with the glass barrel 104. The adhesive 106 is distributed to hold the needle in the narrow area of the glass barrel 104 and above the glass barrel 104.

The glass barrel 104 is the main body of the syringe 100 that might house medication. It is generally made of high-quality glass to ensure transparency, chemical resistance, and/or sterility. The adhesive 106 is generally used to securely bond the needle 108 to the glass barrel 104. Its durable fixation is crucial for maintaining the alignment and position of the needle, ensuring that it remains fixed during use. The needle 108 is essential for the administration of media from inside the barrel. It needs to be precisely aligned with the glass barrel to facilitate accurate and safe delivery of that media.

In the following, further inventive examples of the present disclosure are listed:
1. A method for curing adhesive during assembly of a glass syringe, the method comprising the following steps a.) to d.):
   a.) providing an adhesive, a glass barrel and a needle;
   b.) dispensing the adhesive onto the glass barrel and/or needle;
   c.) arranging the needle onto the barrel;
   d.) curing the adhesive by means of radiation;
   and wherein the method further comprises:
   heating at least one of the glass barrel, needle and adhesive by means of hot air.
2. The method according to example 1, wherein the heating is carried out during and/or after step d.).
3. The method according to one of the examples 1 or 2, wherein the heat-ing is carried out with hot air and UV radiation, preferably provided by a LED.
4. The method according to example 3, wherein the hot air and the UV radiation are used alternately.
5. The method according to any of the preceding examples, wherein the hot air is provided in an angle of incidence in the range 0° to 60°, preferably in the range 15° to 45°, most preferred about 30°.
6. The method according to any of the preceding examples, wherein the radiation is provided in an angle of incidence in the range 0° to 60°, preferably in the range 15° to 45°, most preferred about 30°.
7. The method according to any of the preceding examples, wherein the radiation is provided by a radiation source, and the shortest distance between the radiation source and the adhesive is in the range be-tween 10 mm and 40 mm, preferably in the range 20 mm to 30 mm, most preferably around 26 mm.
8. The method according to any of the preceding examples, wherein the hot air is provided by an air-nozzle, and the shortest distance be-tween the air-nozzle and the adhesive is in the range between 5 mm and 20 mm, preferably in the range 7 mm to 15 mm, most preferably around 10 mm.
9. The method according to any of the preceding examples, wherein the hot air is provided in the vicinity of the glass barrel, needle and/or adhesive with a temperature in the range of between 150 °C to 450°C, preferably be-tween 200 °C and 400°C, more preferably be-tween 220 °C and 300 °C, most preferably around 250 °C.
10. The method according to any of the preceding examples, wherein the wavelength of the radiation is in the range 320 nm to 410 nm, pref-erably in the range 340 nm to 390 nm, most preferably around 365 nm.
11. The method according to any of the preceding examples, wherein the intensity of the radiation is in the range 1500 mW/cm2 to 7000 mW/cm2, preferably in the range 2500 mW/cm² to 6000 mW/cm2, most preferably around 3500 mW/cm2.
12. The method according to any one of the preceding examples, wherein the hot air is provided by an air-nozzle with a flow rate per cm² of the nozzle exit in the range of 60 l/min to 250 l/min, preferably 80 l/min to 200 l/min, more preferably 100 l/min to 150 l/min, most preferably around 125 l/min.
13. The method according to the preceding example 12, wherein the flow rate during the heating process is varied over time.
14. The method according to any one of the preceding examples, wherein the heating is carried out for a duration between 4 and 15 seconds, preferably between 6 and 12 seconds, more preferably between 7 and 10 seconds, most preferably around 8 seconds.
15. The method according to any one of the preceding examples, wherein the radiation is provided for a duration between 4 and 15 seconds, preferably between 6 and 12 seconds, more preferably between 7 and 10 seconds, most preferably around 8 seconds.
16. The method according to any one of the preceding examples, wherein the surface of the adhesive is dry after the heating.
17. The method according to any one of the preceding examples, wherein the temperature during the heating is varied over time.
18. The method according to the preceding example 17, wherein the temperature is steadily decreased or increased over time.
19. The method according to any one of the preceding examples 17 or 18, wherein the temperature variation is done with a gradient ΔT/Δt in the range of from 5 K/min to 50 K/min, preferably from 10 K/min to 40 K/min, more preferably from 20 K/min to 30 K/min.
20. The method according to any one of the preceding examples, wherein the heating is carried out such that the adhesive during the heating has a surface temperature in the range of between 100 °C to 350°C, preferably be-tween 150 °C and 300°C, more preferably between 200 °C and 250 °C.
21. The method according to any one of the preceding examples, wherein before step b.) and/or during step c.) at least one of the glass barrel, needle and adhesive is provided with heat.
22. The method according to any one of the preceding examples, wherein after step b.) and before step d.) the adhesive is pre-cured.
23. A method according to one of the preceding examples, wherein the cur-ing comprises irradiating the adhesive with an LED line array.

### Reference list:

100: syringe
102: direction of syringe movement
104: glass barrel
106: adhesive
108: needle
200: hot air treatment station
202: air nozzle
203: air nozzle exit
300: UV radiation treatment station
302: LED floodlight
a: angle of incidence of the air nozzle
b: distance between air nozzle and adhesive surface
c: angle of incidence of the LED Floodlight
d: distance between LED Floodlight and adhesive surface

## Claims

1. A method for curing adhesive during assembly of a glass syringe, the method comprising the following steps a.) to d.):
a.) providing an adhesive, a glass barrel and a needle;
b.) dispensing the adhesive onto the glass barrel and/or needle;
c.) arranging the needle onto the barrel;
d.) curing the adhesive by means of radiation;
and wherein the method further comprises:
heating at least one of the glass barrel, needle and adhesive by means of hot air.

2. The method according to claim 1, wherein the heating is carried out during and/or after step d.).

3. The method according to one of the claims 1 or 2, wherein the heating is carried out with hot air and UV radiation, preferably provided by a LED, wherein preferably the hot air and the UV radiation are used alternately.

4. The method according to any of the preceding claims, wherein the hot air is provided in an angle of incidence in the range 0° to 60°, preferably in the range 15° to 45°, most preferred about 30°.

5. The method according to any of the preceding claims, wherein the radiation is provided in an angle of incidence in the range 0° to 60°, preferably in the range 15° to 45°, most preferred about 30°.

6. The method according to any of the preceding claims, wherein the radiation is provided by a radiation source, and the shortest distance be-tween the radiation source and the adhesive is in the range between 10 mm and 40 mm, preferably in the range 20 mm to 30 mm, most preferably around 26 mm.

7. The method according to any of the preceding claims, wherein the hot air is provided by an air-nozzle, and the shortest distance between the air-nozzle and the adhesive is in the range between 5 mm and 20 mm, preferably in the range 7 mm to 15 mm, most preferably around 10 mm.

8. The method according to any of the preceding claims, wherein the hot air is provided in the vicinity of the glass barrel, needle and/or adhesive with a temperature in the range of between 150 °C to 450°C, preferably between 200 °C and 400°C, more preferably between 220 °C and 300 °C, most preferably around 250 °C.

9. The method according to any of the preceding claims, wherein the wavelength of the radiation is in the range 320 nm to 410 nm, preferably in the range 340 nm to 390 nm, most preferably around 365 nm.

10. The method according to any of the preceding claims, wherein the intensity of the radiation is in the range 1500 mW/cm² to 7000 mW/cm², preferably in the range 2500 mW/cm² to 6000 mW/cm², most preferably around 3500 mW/cm².

11. The method according to any one of the preceding claims, wherein the hot air is provided by an air-nozzle with a flow rate per cm² of the nozzle exit in the range of 60 l/min to 250 l/min, preferably 80 l/min to 200 l/min, more preferably 100 l/min to 150 l/min, most preferably around 125 l/min, wherein preferably the flow rate during the heating process is varied over time.

12. The method according to any one of the preceding claims, wherein the surface of the adhesive is dry after the heating.

13. The method according to any one of the preceding claims, wherein the temperature during the heating is varied over time, wherein preferably the temperature is steadily decreased or increased over time, wherein preferably the temperature variation is done with a gradient ΔT/Δt in the range of from 5 K/min to 50 K/min, preferably from 10 K/min to 40 K/min, more preferably from 20 K/min to 30 K/min.

14. The method according to any one of the preceding claims, wherein before step b.) and/or during step c.) at least one of the glass barrel, needle and adhesive is provided with heat.

15. The method according to any one of the preceding claims, wherein after step b.) and before step d.) the adhesive is pre-cured.
